# EUROPEAN PATENT APPLICATION

(11) **EP 1 371 641 A1**
(43) Date of publication of application: **17.12.2003**
(21) Application number: 02356104.6
(22) Date of filing: 10.06.2002
(51) Int. Cl.: C07C 403/24

(54) **Process for the formation and isolation of carotenoid crystals**

(71) Applicant: Adisseo France S.A.S., 92160 Antony (FR)
(72) Inventor: Quesnel, Yannick, 1380 Lasne (BE)
(74) Representative: Guerre, Dominique

(57) **Abstract**

A process for the production of carotenoids crystals from a carotenoid-containing plant oleoresin which process comprises (a) a first step of introducing the oleoresin to an organic solvent, an alcohol and a base under reflux temperature of the organic solvent to form a saponification reaction mixture; (b) maintaining said saponification reaction mixture for a period of time sufficient to complete the saponification reaction, thereby producing a saponified product; (c) allowing the resulting saponifiied product to separate into two phases comprising an organic phase and an aqueous phase; (d) separating said phases and extracting the extracting the aqueous phase; (e) distilling said organic phase to obtain the carotenoid crystals.

## Description

The present invention relates to a process for the formation and isolation of carotenoid compounds and in particular to the isolation of xanthophyl crystals from marigols flowers.

Carotenoids are abundant in nature and can be found in fruit, vegetables, leaves, micro organisms and marine organisms. Carotenoids are used as antioxidants, food colorants and in the treatment for cancer prevention. These compounds can be easily extracted from their natural source using classical extraction method such as that disclosed in US patent No. 6191293. This extraction, after solvent removal leads to a crude oleoresin. The resulting carotenoid concentration is not sufficiently high to use the crude oleoresin directly for human consumption.

Alternative chemical processes for the formation, isolation and purification of carotenoids are known, such as the process disclosed in US 5,648,564. When this process is applied on industrial scale, the amount of xanthophyll crystals obtained is modest, typically with a yield of 59% and purity of 79%. Similarly, the process disclosed in WO 01/94279 provides a yield of 80% and a purity 70-51%. Similar problems are encountered in the processes as disclosed in WO 01/28966, ES 2 099 683, WO 99/20587 and. EP 0,732,378.

Furthermore, the processes of the cited prior art appear to produce a large amount of effluent which is heavily polluted with alkaline, usually potassium hydroxide and hydro-miscible organic solvent, usually 1,3-propanediol. The product must therefore be treated which requires an additional expensive step in the industrial process. More over, the isolation of crystallised carotenoids in these processes proceeds via a centrifugation step, probably because of the high viscosity of the crude reaction mixture which involves expensive equipment and high investments.

We have developed a process which overcomes the aforementioned problems encountered in the prior art and accordingly the present invention provides a process for the production of carotenoids crystals from a carotenoid-containing plant oleoresin which process comprises (a) a first step of introducing the oleoresin to an organic solvent, an alcohol and a base under reflux temperature of the organic solvent to form a saponification reaction mixture; (b) maintaining said saponification reaction mixture for a period of time sufficient to complete the saponification reaction, thereby producing a saponified product; (c) allowing the resulting saponifiied product to separate into two phases comprising an organic phase and an aqueous phase; (d) separating said phases and extracting the extracting the aqueous phase; (e) distilling said organic phase to obtain the carotenoid crystals.

The process of the present invention provides an advantage over the known prior art processes in that the resulting product is obtained in a higher yield and has a higher purity. There is no need to purify the crystals obtained.

Furthermore, this invention provides a process for producing pure carotenoids such as carotenes and xanthophylls in all trans conformation. Isomerisation of the cis isomers occurs during the distillation step. Thus, the product obtained directly from the process of the present invention is suitable for human consumption or feed supplementation. A further advantage of the present invention is that the quantity of effluents generated by the process are between 2 and 32 times lower than those of the known processes. The solvents used are distilled off at the end of the process and can be reused, thus providing an environmentally efficient means of producing carotenoids. Furthermore, the equipment used in the process is simple and there is no need of a centrifuge or freeze-dying apparatus, thus providing an attractive option for industrial scale production.

The process of the present invention relates to the extraction of crystals of carotenoids from carotenoid-containing plant. Carotenoids obtained from the process of the present invention include xanthophylls, for example lutein, zeaxanthin, capsorubin, capsanthin, astaxanthin and cathaxanthin; carotenes such as beta-carotene and lycopene. The carotenoid-containing plant are preferably those known to contain a high concentration of the desired carotenoid ester and include marigold flower and berries. The oleresin is extracted by methods known in the art.

In the first step of the present invention, the oleoresin is admixed with an organic solvent, an alcohol and a base to form a saponification reaction mixture. A suitable organic solvent is an organic solvent that is immiscible with water, for example a hydrocarbon such as pentane, hexane, heptane and toluene; ethers such as diisopropylether, methyl ^{ter}butyl ether (MTBE), diethylether; chlorinated solvents such as dichloromethane, dichloroethane, monochlorobenzene (MCB). The concentration of solvent may be from 0.5 to 10 l/kg of oleoresin, preferably from 1 to 4 l/kg d of oleoresin.

As regards the alcohol, this may be an alcohol selected from methanol, ethanol, propanol, isopropanol, ^{ter}butanol and butanol. The preferred alcohol is isopropanol or propanol. The alcohol is present in a concentration of from 0.5 to 10 l/kg oleoresin, preferably from 1 to 4 l/kg of oleoresin.

As regards the base, suitable bases include alkali hydroxide, for example lithium, sodium and potassium hydroxide; an alkali carbonate for example lithium, sodium and potassium carbonate. The preferred base is sodium or potassium hydroxide.

The first step of the process is carried out at the reflux temperature of the solvent. The reactants are maintained at the reflux temperature for a time sufficient to complete the saponification reaction. The resulting saponified product comprises two phases, namely an organic phase and an aqueous phase. The two phases are allowed to separate and the aqueous phase is removed from the reaction system. The remaining organic phase is then distilled to remove traces of the solvents, thereby providing the carotenoid crystals. This step of the process is suitably carried out at a temperature of from 50 to 100°C, preferably from 80 to 85°C. During the distillation step, it is preferred to continuously introduce water into the system to maintain the volume of liquid.

The resulting crystals may be separated by filtration. The filtration may be carried out at a temperature of from 50 to 100°C. The isolated crystals may then be washed. The washing may be carried out with water or a suitable solvent such as acetonitrile, or an alcohol (methanol, ethanol, propanol, isopropanol or the like). Where it is desired to use water, this stage may be carried out at a temperature of from 50 to 100°C, preferably from 80 to 85°C. Where it is desired to a solvent such as acetonitile or an alcohol, this stage may be carried out at a temperature of from minus 5 to 40°C, preferably from 20 to 25°C.

The amount of water may suitable be from 1 to 20 l/kg, preferably from 5 to 10 l/kg.

The resulting crystals are obtained with a high purity, typically between 85 and 99%, generally greater than 95%. The yield of product is typically between 40 and 90%; generally between 70 and 80%.

The present invention will now be described in detail with reference to the following examples:

### Example 1:

72.8g of Marigold oleoresin (obtained from Naturex, France, with a content of 128g of carotenoids per kilogram of oleoresin) were dissolved in 150 ml of hexane and 150 ml of isopropanol under reflux. 150 ml of aqueous sodium hydroxyde (30%wt) was then introduced and the crude reaction mixture was maintained under reflux under strong agitation and a nitrogen atmosphere until the saponification was complete (2.5h, complexion >97% determined by High Performance Liquid Chromatography (HPLC). At this point, the agitation was stopped and the mixture rapidly separated into two phases (upper organic phase and lower aqueous phase). The lower phase aqueous phase was removed. The organic solvents (hexane & isopropanol) were distilled off while introducing 500 ml of pre-heated water (90°C) with a input flow approximately equal to the output flow so that the total volume of the liquid phase did not exceed 600ml. At the end of the distillation the crude mixture, containing an aqueous liquid phase and the crystallised carotenoid, was filtered to isolate the crystallised carotenoid. The filter cake was washed with three aliquots of 150ml of warm water, followed by three aliquots of 150ml of room temperature acetonitrile and then by three aliquots of 150ml of room temperature room temperature hexane. The residual traces of solvent were removed under reduced pressure at room temperature. 6.76g of orange/red crystals of pure xanthophyll were collected (Yield : 72.3% based on total carotenoids). The crystals were analysed by HPLC : 91.2% of all trans lutein, 8.8% of all trans zeaxanthine, cis isomers and other carotenoids were not detected, total carotenoids yield was more than 95%).

### Example 2 :

82.5g of Marigold oleoresin (obtained from Plant Lipids Limited, India, with a content of 100g of carotenoids per kilogram of oleoresin) were dissolved in 150 ml of hexane and 150 ml of isopropanol under reflux. Then 150 ml of aqueous sodium hydroxyde (30%wt) was introduced and the crude reaction mixture was maintained under reflux under strong agitation and N₂ atmosphere until the saponification was complete (2.5h, complexion >97% determined by HPLC). At this point, the agitation was stopped and the mixture rapidly separated into 2 phases (upper phase : organic phase, lower phase : aqueous phase). The lower phase (aqueous phase) was removed. The organic solvents (i.e. hexane & isopropanol) were distilled off while introducing 500 ml of pre-heated water (90°C) with a input flow approximately equal to the output flow so that the total volume of the liquid phase did not exceed 600ml. At the end of the distillation the crude mixture (containing an aqueous liquid phase and crystallised carotenoid) was filtered to isolate the crystallised carotenoids. The filter cake was washed with three aliquots of 150ml of warm water, followed by three aliquots of 150ml of room temperature ethanol. The residual traces of solvent were removed under reduced pressure at room temperature. 6.45g of orange/red crystals of pure Xanthophyll were collected (Yield : 78.2% based on total carotenoids). This crystals were analysed by HPLC : 91.2% of all trans Lutein, 8.8% of all trans Zeaxanthine, cis isomers and other carotenoids were not detected, total carotenoids yield was more than 95%).

### Example 3 :

56.1g of Marigold oleoresin (obtained from Naturex, France, with a content of 128g of carotenoids per kilogram of oleoresin) was dissolved in 150 ml of hexane and 150 ml of isopropanol under reflux. Then 150 ml of aqueous sodium hydroxyde (30%wt) was introduced and the crude reaction mixture was maintained under reflux under strong agitation and nitrogen atmosphere until the saponification was complete (2.5h, complexion >97% determined by HPLC). At this point, the agitation was stopped and the mixture rapidly separated into two phases, upper organic phase and lower aqueous phase. The lower aqueous phase was removed. The organic was slowly poured into 500 ml of pre-heated water (95°C) and the organic solvents immediately distilled out, the input flow was adjusted to keep an internal volume below 600ml. At the end of the addition, heating was kept until distillation of organic solvents was finished, then, carotenoid crystals were collected by filtration. The filter cake was washed with three aliquots of 150ml of warm water, followed by three aliquots of 150ml of room temperature hexane. The residual traces of solvent were removed under reduced pressure at room temperature. 3.5g of orange/red crystals of pure Xanthophyll were collected (Yield : 51% based on total carotenoids, 92% of carotenoids contents). This crystals were analysed by HPLC : 93% of all trans Lutein, 7% of all trans Zeaxanthine, cis isomers and other carotenoids were not detected, total carotenoids yield was more than 95%).

### Example 4-17 :

Commercially available Marigold Oleoresin was dissolved in 10 ml of heptane at 45°C, 2.5 ml of butanol and 2ml of aqueous sodium hydroxide (30%wt). The reaction mixture was kept under agitation and inert atmosphere for 5 hours. The, agitation was stopped and the mixture rapidly separated into 2 phases. The separation of the two phases and treatment thereafter was carried out as in Example 1. The yield of product is reported in Table 1 below.

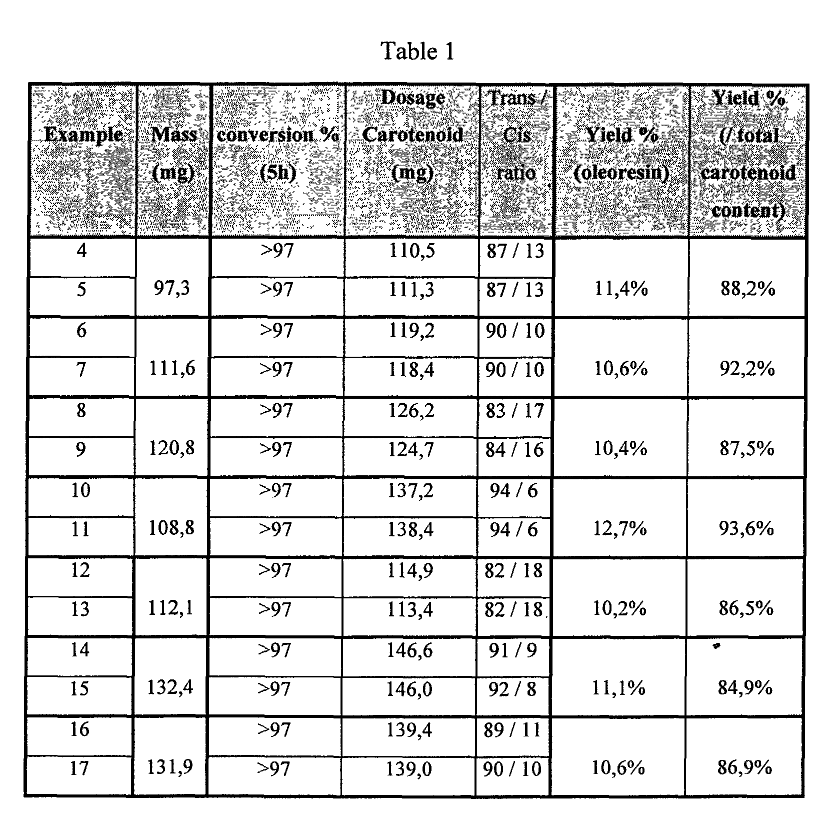

### Example 18-23 :

1g of oleoresin (obtained from Plant Lipids Limited, 100g of carotenoids per kg) was dissolved in the various solvents as detailed in Table 2 below., alcohol was added, followed by 3 ml of alkali hydroxide. The reaction mixture was kept under agitation and inert atmosphere until completion.

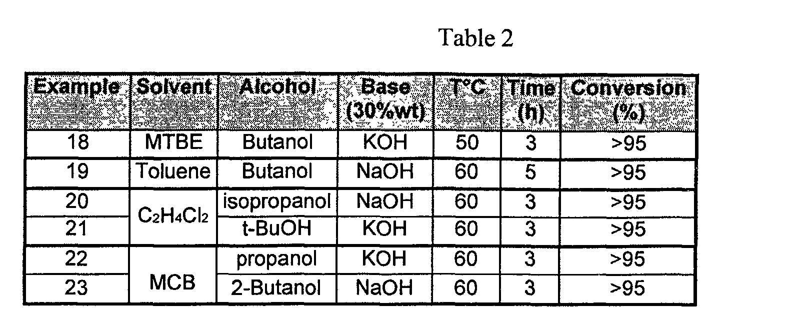

## Claims

1. A process for the production of carotenoids crystals from a carotenoid-containing plant oleoresin which process comprises (a) a first step of introducing the oleoresin to an organic solvent, an alcohol and a base under reflux temperature of the organic solvent to form a saponification reaction mixture; (b) maintaining said saponification reaction mixture for a period of time sufficient to complete the saponification reaction, thereby producing a saponified product; (c) allowing the resulting saponifiied product to separate into two phases comprising an organic phase and an aqueous phase; (d) separating said phases and extracting the extracting the aqueous phase; (e) distilling said organic phase to obtain the carotenoid crystals.

2. A process as claimed in claim 1 in which the organic solvent is an organic solvent that is immiscible with water.

3. A process as claimed in claim 2 in which the organic solvent is a hydrocarbon, an ether or a chlorinated solvent.

4. A process as claimed in claim 3 in which the organic solvent is a hydrocarbon selected from such as pentane, hexane, heptane and toluene.

5. A process as claimed in any one of the preceding claims in which the alcohol is selected from methanol, ethanol, propanol, isopropanol, ^{ter}butanol and butanol.

6. A process as claimed in any one of the preceding claims in which the base is selected from alkali hydroxide or alkali carbonate.

7. A process as claimed in claim 6 in which the base is lithium, sodium or potassium hydroxide.

8. A process as lciamed in any one of the preceing claims in which water is continuously introduced water into the system during the distillation of the organic phase.
